(19) Europäisches Patentamt
European Patent Office
Office européen des brevets

(11) EP 1 685 091 B1

(12) EUROPÄISCHE PATENTSCHRIFT

(45) Veröffentlichungstag und Bekanntmachung des Hinweises auf die Patenterteilung:
**19.05.2010 Patentblatt 2010/20**

(21) Anmeldenummer: **04764752.4**

(22) Anmeldetag: **02.09.2004**

(51) Int Cl.:
*C07C 211/51* (2006.01) *C07C 217/80* (2006.01)
*C07C 215/74* (2006.01) *C07D 213/38* (2006.01)
*C07C 255/58* (2006.01) *C07D 333/20* (2006.01)
*C07D 307/52* (2006.01) *C07D 209/14* (2006.01)
*A61K 8/41* (2006.01) *A61K 8/49* (2006.01)
*A61Q 5/10* (2006.01)

(86) Internationale Anmeldenummer:
**PCT/EP2004/009794**

(87) Internationale Veröffentlichungsnummer:
**WO 2005/051889 (09.06.2005 Gazette 2005/23)**

(54) **M-DIAMINOBENZOLE UND DEREN SÄUREADDUKTE SOWIE DEREN VERWENDUNG IN FÄRBEMITTELN**

M-DIAMINOBENZOLES AND THE ACIDIC ADDUCTS THEREOF AND USE THEREOF IN COLOURING AGENTS

M-DIAMINOBENZ NES ET LEURS PRODUITS D'ADDITION D'ACIDE, AINSI QUE LEUR UTILISATION DANS DES COLORANTS

(84) Benannte Vertragsstaaten:
**AT BE BG CH CY CZ DE DK EE ES FI FR GB GR HU IE IT LI LU MC NL PL PT RO SE SI SK TR**

(30) Priorität: **06.11.2003 DE 10351842**

(43) Veröffentlichungstag der Anmeldung:
**02.08.2006 Patentblatt 2006/31**

(73) Patentinhaber: **THE PROCTER & GAMBLE COMPANY**
**Cincinnati, Ohio 45202 (US)**

(72) Erfinder:
- **GÖTTEL, Otto**
  **CH-1723 Marly (CH)**
- **HAYOZ, André**
  **CH-1724 Senèdes (CH)**
- **MORAND, Emmanuel**
  **CH-1752 Villars-sur-Glâne (CH)**

(74) Vertreter: **Hirsch, Uwe Thomas M.H. et al**
**Procter & Gamble Service GmbH**
**Patent Department**
**Berliner Allee 65**
**64274 Darmstadt (DE)**

(56) Entgegenhaltungen:
**DE-A1- 2 852 156** **DE-A1- 3 229 973**
**US-A- 3 232 995** **US-A- 5 518 507**
**US-B1- 6 461 389**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 685 091 B1

Printed by Jouve, 75001 PARIS (FR)

## Beschreibung

Die Erfindung betrifft Oxidationsfarbstoffvorstufen sowie diese Verbindungen enthaltenden Mittel zur Färbung von keratinhaltigen Fasern, insbesondere von menschlichen Haaren.

Auf dem Gebiet der traditionellen Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels. Von besonderer Bedeutung sind Haarfärbemittel zur Färbung im Naturtonbereich, durch eine geeignete Kombination von Entwicklern und Kupplern lassen sich auch modische Töne erzielen.
An Farbstoffe, die zur Färbung menschlicher Haare vorgesehen sind, werden besondere Anforderungen gestellt. Die Farbstoffe sollten einerseits Färbungen in der gewünschten Farbe und Intensität ermöglichen, andererseits müssen sie in toxikologischer wie dermatologischer Hinsicht unbedenklich und nicht sensibilisierend sein.

Die Anforderungen an Farbstoffe, die zur Erzielung modischer Töne vorgesehen sind, können sich signifikant von denen zur Abdeckung des Naturtonbereichs unterscheiden. Ein Hauptkriterium ist beispielsweise die Farbreinheit, die sich in der Brillanz des Färbeergebnisses widerspiegelt. Insbesondere im Blaubereich wurden bisher noch keine Kombinationen gefunden, die es erlaubten, zusammen mit den häufig eingesetzten p-p-Phenylendiaminen (beispielsweise p-Phenylendiamin, p-Toluylendiamin und 2,5-Diamino-(2'-hydroxyethyl)-benzol) sehr reine Blautöne zu erzeugen. Übliche Blaukuppler wie zum Beispiel 2,4-Diamino-phenoxyethanol, 5-((2-Hydroxyethyl)amino)-2-methoxy-anilin oder 1,3-Di(2,4-diaminophenoxy)-propan können keine brillanten Blautöne liefern, da sie einen erheblichen Rotanteil verursachen. Dieser Rotanteil ist beispielsweise im L*a*b*-Farbsystem durch erhöhte a*-Werte zu erkennen.

Aus der US 6 461 389 B1 sind Oxidationshaarfärbemittel bekannt, welche spezielle kationische Kupplerverbindungen enthalten.

Es bestand daher die Aufgabe, neue Kupplerverbindungen zur Verfügung zu stellen, welche -insbesondere in Kombination mit den häufig verwendeten p-Phenylendiaminen- reinere Blautöne liefern, und Oxidationshaarfärbemittel mit hoher Stabilität gegen externe Einflüsse, insbesondere gegenüber häufigem Waschen und Sonnenlicht, ermöglichen.

Überraschenderweise wurde nun gefunden, dass bestimmte m-Diamino-benzol-Derivate die gestellten Aufgaben in hervorragender Weise erfüllen.

Ein Gegenstand der vorliegenden Erfindung sind daher m-Diaminobenzole, welche ausgewählt sind aus der Gruppe bestehend aus

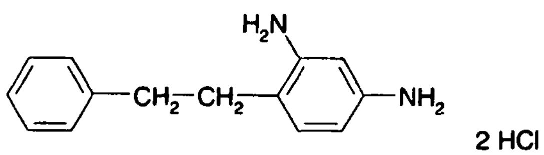

1,3-Diamino-4-(2-phenylethyl)-benzol-dihydrochlorid (1a)

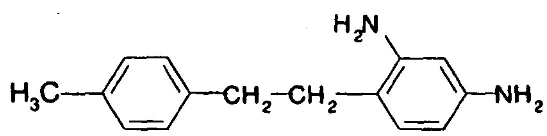

1,3-Diamino-4-[2-(4-methylphenyl)ethyl]-benzol (1b)

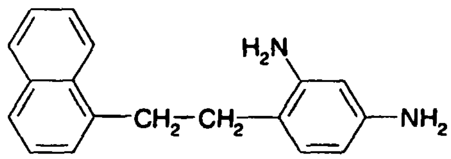

2 HCl

1,3-Diamino-4-[2-(1-naphthyl)ethyl]-benzol-dihydrochlorid (1c)

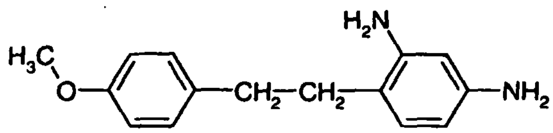

2 HCl

1,3-Diamino-4-[2-(4-methoxyphenyl)ethyl]-benzol-dihydrochlorid (1d)

1,3-Diamino-4-[2-(4-methoxyphenyl)ethyl]-benzol (1d')

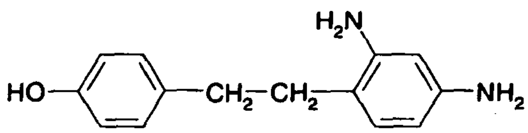

2 HCl

1,3-Diamino-4-[2-(4-hydroxyphenyl)ethyl]-benzol-dihydrochlorid (1e)

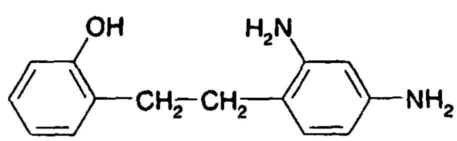

1,3-Diamino-4-[2-(2-hydroxyphenyl)ethyl]-benzol (1f)

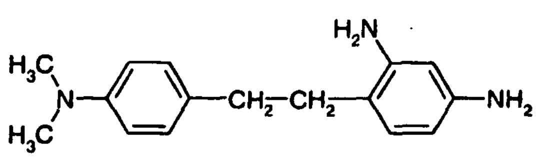

3 HCl

1,3-Diamino-4-[2-(4-(dimethylamino)phenyl)ethyl]-benzol-trihydrochlorid (1g)

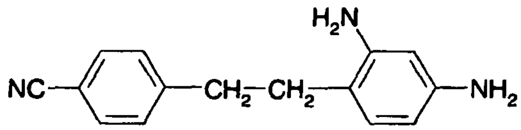

2 HCl

4-[2-(4-Cyanophenyl)ethyl]-1,3-diamino-benzol-dihydrochlorid (1h)

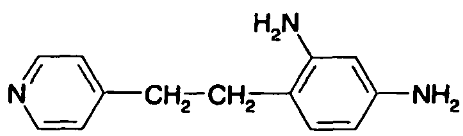

3 HCl

1,3-Diamino-4-[2-(4-pyridinyl)ethyl]-benzol-trihydrochlorid (1i)

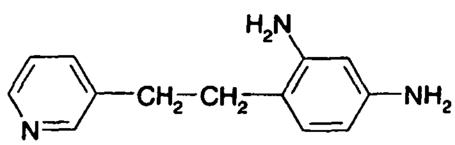

3 HCl

1,3-Diamino-4-[2-(3-pyridinyl)ethyl]-benzol-trihydrochlorid (1k)

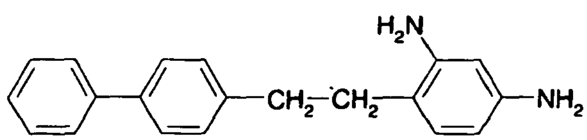

2 HCl

1,3-Diamino-4-[2-(4-biphenyl)ethyl]-benzol-dihydrochlorid (1l)

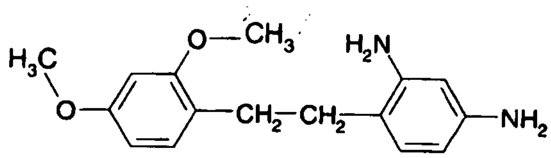

1,3-Diamino-4-[2-(2,4-dimethoxyphenyl)ethyl]-benzol (1m)

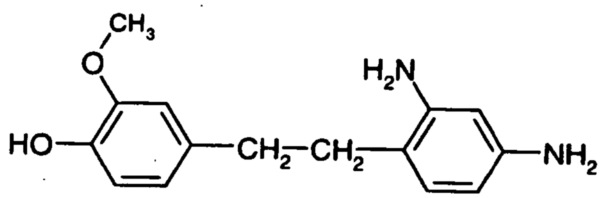

1,3-Diamino-4-[2-(4-hydroxy-3-methoxyphenyl)ethyl]-benzol (1n)

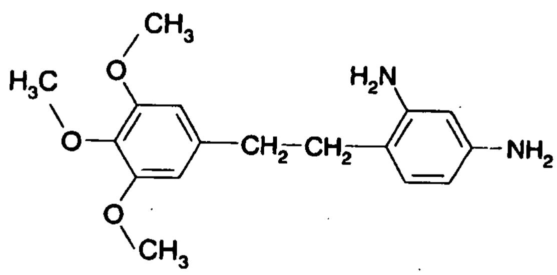

1,3-Diamino-4-[2-(3,4,5-trihydroxyphenyl)ethyl]-benzol (1o)

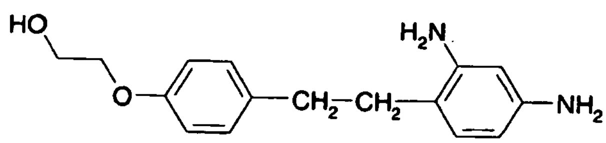

1,3-Diamino-4-[2-(4-(2-hydroxyethoxy)phenyl)ethyl]-benzol (1p)

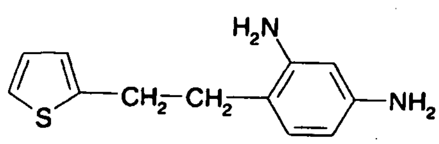

1,3-Diamino-4-[2-(2-thienyl)ethyl]-benzol (1q)

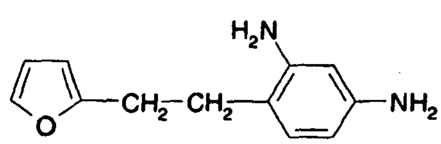

1,3-Diamino-4-[2-(2-furyl)ethyl]-benzol (1r)

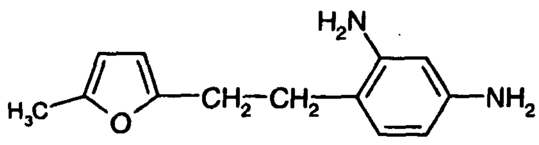

1,3-Diamino-4-[2-(5-methyl-2-furyl)ethyl]-benzol (1s)

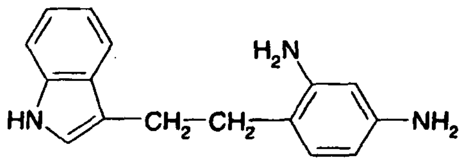

1,3-Diamino-4-[2-(1H-indol-3-yl)ethyl]-benzol (1t)

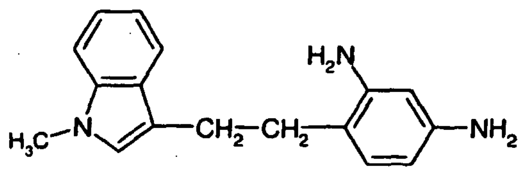

1,3-Diamino-4-[2-(1-methyl-1H-indol-3-yl)ethyl]-benzol (1u)

und

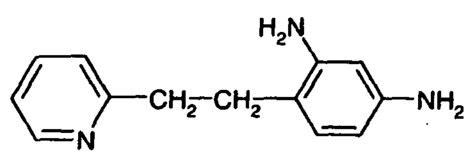

3 HCl

1,3-Diamino-4-[2-(2-pyridinyl)ethyl]-benzol-trihydrochlorid (1v).

Die Herstellung der erfindungsgemässen m-Diaminobenzole (I) erfolgt vorzugsweise unter Knoevenagel-Bedingungen durch Kondensation von Aldehyden der allgemeinen Formel (III) mit 2,4-Dinitrotoluol (IV) zu den Stilbenen (V). Zur Kondensation sei insbesondere auf die beiden Literaturen S. B Lokhande, D. W. Rangnekar, Ind. J. Chem. 25B, 485-8 (1986) sowie B. G. Tiemann e. a., Chem. Mater 1990, 2, 690-695 verwiesen. Durch eine vollständige Reduktion der Nitrogruppen wie. auch der Stilben-Doppelbindung hat man den Zugang zu den m-Phenylendiaminen (I), die als freie Basen oder vorzugsweise als Säureaddukte isoliert werden können.

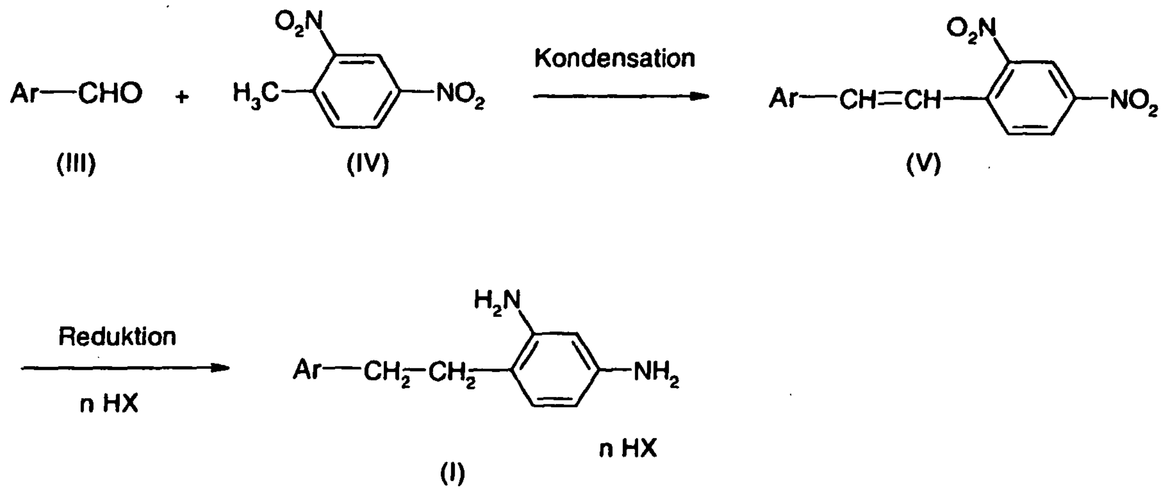


(III) (IV) (V) (I)

Die erfindungsgemässen m-Diaminobenzole eignen sich hervorragend als Farbstoff-Vorstufen im oxidativen System zum Färben von natürlichen und synthetischen Fasern.

Ein weiterer Gegenstand der vorliegenden Erfindung ist daher die Verwendung der erfindungsgemässen m-Diaminobenzole zur oxidativen Färbung von Fasern, insbesondere keratinischen Fasern, wie zum Beispiel Wolle oder Haare und insbesondere menschliche Haare.

Ein weiterer Gegenstand der vorliegenden Erfindung ist ein Mittel zur oxidativen Färbung von Fasern, insbesondere keratinischen Fasern, wie zum Beispiel Wolle oder Haare und insbesondere menschliche Haare, das in einer geeigneten Farbträgermasse mindestens ein erfindungsgemässes m-Diaminobenzol enthält.

Die erfindungsgemässen m-Diaminobenzole können hierbei alleine oder in Kombination mit anderen Entwicklersubstanzen und/oder Kupplersubstanzen, eingesetzt werden, wobei die Einsatzmenge der erfindungsgemässen m-Diaminobenzole etwa 0,01 bis 20 Gewichtsprozent, vorzugsweise 0,1 bis 10 Gewichtsprozent, beträgt.

Als Entwicklersubstanzen, die sich in hervorragender Weise zur Kombination mit den erfindungsgemäßen Verbindungen in einer oxidativen Formulierung eignen, können beispielsweise Paraphenylendiamin-Derivate, Paraaminophenol-Derivate und 4,5-Diaminopyrazol-Derivate sowie deren Salze genannt werden. Insbesondere sind die folgenden Verbindungen zu nennen:

1,4-Diaminobenzol (p-Phenylendiamin), 1,4-Diamino-2-methyl-benzol (p-Toluylen-diamin), 1,4-Diamino-2,6-dimethyl-benzol, 1,4-Diamino-2,5-dimethyl-benzol, 1,4-Diamino-2,3-dimethyl-benzol, 2-Chlor-1,4-diamino-benzol, 4-Phenylamino-anilin, 4-Dimethylamino-anilin, 4-Diethylaminoanilin, 4-[Di(2-hydroxyethyl)amino]-anitin, 4-[(2-Methoxyethyl)amino]-anilin, 4-[(3-Hydroxypropyl)-amino]-anilin, 1,4-Diamino-2-(2-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylaminophenol, 4-Amino-2-(aminomethyl)-phenol, 4-Amino-2-[(2-hydroxyethyl)-amino]methyl-phenol, 4-Amino-2-(methoxy-methyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methyl-phenyl)methyl]-1H-pyrazol, 1-[(4-Chlorphenyl)-methyl]-4,5-diamino-1H-pyrazol, 4,5-Diamino-1-methyl-1H-pyrazol, 4,5-Diamino-3-methyl-1-phenyl-1H-pyrazol, 4,5-Diamino-1-(2-hydroxyethyl)-3-methyl-1H-pyrazol, 1,2-Bis-(4,5-diamino-1H-pyrazol-1-yl)-ethan, 1,4-Bis-(4,5-diamino-pyrazol-1-yl-methyl)-benzol, 4,5-Diamino-1-(2-methylphenyl)-1H-pyrazol, 4,5-Diamino-1-(3-methylphenyl)-1H-pyrazol, 4,5-Diamino-1-(4-methylphenyl)-1H-pyrazol, 4,5-Diamino-1-(2,4-dimethylphenyl)-1H-pyrazol, 4,5-Diamino-1-(2,5-dimethylphenyl)-1H-pyrazol, 4,5-Diamino-1-(2-ethylphenyl)-1H-pyrazol, 4,5-Diamino-1-(4-isopropylphenyl)-1H-pyrazol, 4,5-Diamino-1-(4-methoxyphenyl)-1H-pyrazol, 1-(4-Amino-phenyl)-4,5-diamino-1H-pyrazol, 1-(4-Chlorphenyl)-4,5-diamino-1H-pyrazol, 4,5-Diamino-1-(2-pyridinyl)-1H-pyrazol, 2-Amino-phenol, 2-Amino-6-methyl-phenol und 2-Amino-5-methyl-phenol, oder deren Salze.

Als geeignete zusätzliche Kupplersubstanzen, die zur Erzeugung bestimmter Nuancen in Kombination mit den erfindungsgemäßen Verbindungen eingesetzt werden können, sind zu nennen:

N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol,1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methylphenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

Weiterhin können in dem erfindungsgemäßen Färbemittel auch direktziehende anionische, kationische oder neutrale Farbstoffe enthalten sein. Zu den bevorzugten anionischen Farbstoffen zählen beispielsweise 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl0316; Acid Yellow No. 1; Food Yellow No. 1), 2-(Indan-1,3-dion-2-yl)chinolin-x,x-sulfonsäure (Gemisch aus Mono- und Disulfonsäure) (C147005;D&C Yellow No. 10; Food Yellow No. 13, Acid Yellow No. 3), 5-Hydroxy-1-(4-sulfophenyl)-4-[(4-sulfophenyl)-azo]pyrazol-3-carbonsäure-trinatriumsalz (C119140; Food Yellow No. 4; Acid Yellow No. 23), 9-(2-Carboxyphenyl)-6-hydroxy-3H-xanthen-3-on (C145350; Acid Yellow No. 73; D&C Yellow No. 8), 5-[(2,4-Dinitrophenyl)-amino]-2-phenylamino-benzolsulfonsäure-natriumsalz (C110385; Acid Orange No. 3), 4-[(2,4-Dihydroxyphenyl)azo]-benzolsulfonsäure-mononatriumsalz (C114270; Acid Orange No. 6), 4-[(2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2,4-naphthalindisulfonsäure-trinatriumsalz (Cl16255; Ponceau 4R; Acid Red No. 18), 3-Hydroxy-4-[(4-sulfonaphth-1-yl)azo]-2,7-naphthalin-disulfonsäure-trinatriumsalz (Cl16185; Acid Red No. 27), 8-Amino-1-hydroxy-2-(phenylazo)-3,6-naphthalin-disulfonsäure-dinatriumsalz (Cl17200; Acid Red No. 33), 5-(Acetylamino)-4-hydroxy-3-[(2-methylphenyl)azo]-2,7-naphthalin-disulfonsäure-dinatriumsalz (Cl18065; Acid Red No. 35), 2-(3-Hydroxy-2,4,5,7-tetraiod-dibenzopyran-6-on-9-yl)-benzoesäure-dinatriumsalz (C145430;Acid Red No. 51), N-[6-(Diethylamino)-9-(2,4-disulfophenyl)-3H-xanthen-3-yliden]-N-ethylethanaminium-hydroxid, inneres Salz, Natriumsalz (Cl45100; Acid Red No. 52), 8-[(4-(Phenylazo)-phenyl)azo]-7-naphthol-1,3-disulfonsäure-dinatriumsalz (Cl27290; Acid Red No. 73), 2',4',5',7'-Tetrabrom-3',6'-dihydroxyspiro[isobenzofuran-1(3H),9'-[9H]xanthen]-3-on-dinatriumsalz (Cl45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (Cl45410; Acid Red No. 92), 3',6'-Dihydroxy-4',5'-düodospiro[isobenzofuran-1(3H),9'(9H)-xanthen)-3-on-dinatriumsalz (Cl45425; Acid Red No. 95), (2-Sulfophenyl)di[4-(ethyl((4-sulfophenyl)methyl)amino)phenyl]-carbenium-dinatriumsalz, betain (Cl42090; Acid Blue No. 9; FD&C Blue No. 1), 1,4-Bis[(2-sulfo-4-methylphenyl)amino]-9,10-anthrachinon-dinatriumsalz (Cl 61570; Acid Green No. 25), Bis[4-(dimethylamino)phenyl]-(3,7-disulfo-2-hydroxynaphth-1-yl)-carbenium-inneres Salz, mononatriumsalz (Cl44090; Food Green No. 4; Acid Green No. 50), Bis[4-(diethylamino)phenyl](2,4-disulfophenyl)carbenium-inneres salz, Natriumsalz (2:1) (Cl42045; Food Blue No. 3; Acid Blue No. 1), Bis[4-(diethylamino)phenyl](5-hydroxy-2,4-disulfophenyl)carbenium-inneres salz, Calciumsalz (2:1) (Cl42051; Acid Blue No. 3), 1-Amino-4-(cyclohexylamino)-9,10-anthrachinon-2-sulfonsäure-natriumsalz (Cl62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (Cl73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (Cl45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis{ 3-nitro-4-[(4-phenylamino)-3-sulfo-phenylamino]-phenyl }-sulfon (Cl10410; Acid Brown No. 13), 5-Amino-4-hydroxy-6-[(4-nitrophenyl)azo]-3-(phenylazo)-2,7-naphthalindisulfonsäure-dinatriumsalz (C120470; Acid Black No. 1), 3-Hydroxy-4-[(2-hydroxynaphth-1-yl)azo]-7-nitro-1-naphthalin-sulfonsäure-chromkomplex (3:2)

(CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl) azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure natriumsalz Chrom-Komplex (Acid Red No. 195).

Zum besseren Farbausgleich und zur Erzeugung von speziellen Nuancen haben sich insbesondere folgende nichtionische Farbstoffe bewährt: 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)-mino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)-amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)-amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitro-diphenylamin (HC Red No. 1), 1-Amino-4-[di(2-hydroxyethyl)aminoJ-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitrophenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitro-pyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methyl-amino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8), 1-[(3-Amino-propyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin und 2-((4-(Acetyl-amino)phenyl)-azo)-4-methylphenol (CI11855; Disperse Yellow No. 3).

Aus der Gruppe der direktziehenden Farbstoffe besonders zu erwähnen sind auch 2-Amino-4,6-dinitrophenol, 2-Ethylamino-4,6-dinitrophenol, 2-[(2-Hydroxyethyl)amino]-4,6-dinitrophenol und Farbstoffe der allgemeinen Formel (VI),

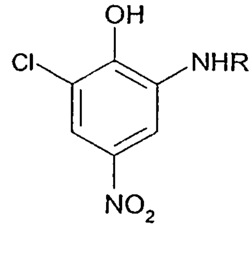

(VI)

worin R Wasserstoff, Methyl, Ethyl oder Hydroxyethyl bedeutet.

Die Gesamtkonzentration an Entwicklersubstanzen und Kupplersubstanzen beträgt in dem gebrauchsfertigen Färbemittel vorzugsweise etwa 0,01 bis 10 Gewichtsprozent, insbesondere etwa 0,1 bis 6 Gewichtsprozent, während die Gesamtkonzentration an direktziehenden Farbstoffen bei 0,1 bis 10 Gewichtsprozent, insbesondere 0,1 bis 5 Gewichtsprozent, liegt.

Darüber hinaus können in der Farbträgermasse noch Antioxidantien, Parfümöle, Komplexbildner, Netzmittel, Emulgatoren, Penetrationsmittel, Puffersysteme, Konservierungsstoffe, Verdicker, Pflegestoffe und andere kosmetische Zusätze enthalten sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze für Lösungen, Cremes, Emulsionen oder Gele sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionischen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet. Bezogen auf die Farbträgermasse sind dies zum Beispiel Netzmittel und Emulgatoren in Konzentrationen von etwa 0,5 bis 30 Gewichtsprozent, Verdicker in einer Menge von etwa 0,1 bis 25 Gewichtsprozent und die Pflegestoffe in einer Konzentration von etwa 0,1 bis 5 Gewichtsprozent.

Das gebrauchsfertige erfindungsgemäße Färbemittel wird durch Mischen der Farbträgermasse mit einem geeigneten Oxidationsmittel unmittelbar vor der Anwendung hergestellt. Prinzipiell ist es auch möglich, dass die Oxidation durch Luft -mit oder ohne enzymatische Unterstützung- erfolgt.

Als Oxidationsmittel kommen hauptsächlich Wasserstoffperoxid oder dessen Additionsverbindungen an Harnstoff, Melamin oder Natriumbromat in Form einer 1 bis 12-prozentigen, vorzugsweise 6-prozentigen, wäßrigen Lösung in Betracht, wobei Wasserstoffperoxid besonders bevorzugt wird.

Die Farbträgermasse und das Oxidationsmittel werden hierbei im Gewichtsverhältnis von etwa 5 zu 1 bis etwa 1 zu 3 miteinander vermischt, wobei ein Gewichtsverhältnis von 1 zu 1 bis 1 zu 2 besonders bevorzugt ist.

Des pH-Wert des gebrauchsfertigen erfindungsgemäßen Färbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis bestimmt wird. Der pH-Wert des fertigen Färbemittels beträgt etwa 3 bis 11, vorzugsweise 6 bis 10,5.

Für die Einstellung des jeweiligen pH-Wertes der Farbträgermasse und des Oxidationsmittels können je nach dem gewünschten pH-Wert verdünnte organische oder anorganische Säuren, wie zum Beispiel Phosphorsäure, Ascorbinsäure und Milchsäure, oder Alkalien wie Monoethanolamin, Triethanolamin, 2-Amino-2-methyl-1-propanol, Ammoniak, Natronlauge, Kalilauge, Aminosäuren oder Tris(hydroxymethyl)amino-methan, verwendet werden.

Nach der Vermischung der Farbträgermasse mit dem Oxidationsmittel wird eine für die Färbebehandlung ausreichende Menge, im allgemeinen etwa 60 bis 200 Gramm, des erhaltenen gebrauchsfertigen Oxidationsfärbemittels auf die Faser aufgetragen.

Man läßt das erfindungsgemäße Färbemittel etwa 10 bis 45 Minuten bei etwa 15 bis 50 Grad Celsius, vorzugsweise 30 Minuten bei 40 Grad Celsius, auf die Faser einwirken, und spült dann die Faser mit Wasser aus. Gegebenenfalls wird im Anschluß an diese Spülung mit einem Shampoo gewaschen und eventuell mit einer verdünnten schwachen organischen Säure, wie zum Beispiel Zitronensäure oder Weinsäure nachgespült. Anschließend wird die Faser getrocknet.

Die nachfolgenden Beispiele sollen den Gegenstand näher erläutern, ohne ihn jedoch auf diese Beispiele zu beschränken.

## Beispiele

### Beispiel 1: Herstellung von 1,3-Diamino-4-(2-phenylethyl)-benzol-dihydrochlorid

0,4 g 2,4-Dinitro-1-[(E)-2-phenylethenyl]benzol, hergestellt nach S. B Lokhande, D. W. Rangnekar, Ind. J. Chem. 25B, Seite 485-488 (1986), werden in 40 ml Ethanol gelöst, mit 50 mg Pd/C 10%ig versetzt und 4 Stunden lang bei 9 bar Wasserstoffdruck hydriert. Anschließend wird der Katalysator durch Filtrieren über Kieselgur entfernt und das Filtrat mit 10 ml 1,8molarer alkoholischer Salzsäure versetzt. Durch Zugabe von Essigester nach Maßgabe der Kristallisation erhält man 0,38 g Produkt mit einem Schmelzpunkt von >250 ˚C (Zers.).

FAB-MS: 213 $[M+H]^+$, 100%

**Beispiel 2: Herstellung von 1,3-Diamino-4-[2-(4-methylphenyl)ethyl]-benzol**

1,4 g 1,3-Dinitro-4-[2-(4-methylphenyl)ethenyl]-benzol, hergestellt nach S. Saravanan und P. Srinivasan, Synth. Commun. 31 (6), Seite 823-826 (2001), werden in 50 ml Ethanol an 0,2 g Pd/C 10%ig 4 Stunden lang bei 8 bar Wasserstoffdruck hydriert. Der Katalysator wird anschließend abfiltriert und der Rückstand zur Trockene eingedampft. Man erhält ein bräunliches Öl, das nach der Zugabe von 10 ml Ether auskristallisiert. Ausbeute: 1,1 g gelbliches Produkt.
$^1$H-NMR (DMSO-$d_6$): δ = 7,15 ppm (d, $^3J_{HH}$ = 7,8 Hz, 2H); 7,08 ppm (d, $^3J_{HH}$ = 7,8 Hz, 2H); 6,57 ppm (d, $^3J_{HH}$ = 7,9 Hz, 1H); 5,91 ppm (d, $^4J_{HH}$ = 2,1 Hz, 1H); 5,79 ppm (dd, $^3J_{HH}$ = 7,9 Hz, $^4J_{HH}$ = 2,1 Hz, 1H); 4,51 ppm (s breit, 4H); 2,65 ppm (m, 2H); 2,55 ppm (m, 2H); 2,27 ppm (s, 3H).

**Beispiel 3: Herstellung von 1,3-Diamino-4-[2-(4-methoxyphenyl)-ethyl]-benzol-dihydrochlorid**

2,86 g 1,3-Dinitro-4-[2-(4-hxdroxyphenyl)ethenyl]-benzol, hergestellt nach S. Saravanan und P. Srinivasan, Synth. Commun. 31 (6), Seite 823-826 (2001), werden in 50 ml Ethanol an 0,3 g Pd/C 10%ig 2 Stunden lang bei 8 bar Wasserstoffdruck hydriert. Der Katalysator wird anschließend abfiltriert, das Filtrat mit 30 ml 1,8 molarer ethanolischer Salzsäure versetzt und anschließend zur Trockene eingedampft. Man erhält ein bräunliches Öl, das nach der Zugabe von 30 ml Essigsäureethylester kristallisiert. Ausbeute: 2,7 g beiges Produkt.
$^1$H-NMR (DMSO-$d_6$): δ = 9,33 ppm (s, sehr breit, 6H); 7,35-7, 28 (Signalüberlagerung, 2H); 7,10 ppm (d, $^3J_{HH}$ = 8,3 Hz, 2H + Signalüberlagerung 1H); 6,70 ppm (d, $^3J_{HH}$ = 8,3 Hz, 2H); 2,85 ppm (m, 2H); 2,79 ppm (m, 2H).

**Beispiel 4: Herstellung von 1,3-Diamino-4-[2-(4-methoxyphenyl)-ethyl]-benzol**

1 g 1,3-Diamino-4-[2-(4-methoxyphenyl)ethyl]-benzol-dihydrochlorid aus Beispiel 3 wird in 50 ml Wasser mit gesättigter Natriumcarbonatlösung bis pH 8 versetzt. Man saugt ab, wäscht mit wenig kaltem Wasser nach und trocknet im Vakuum über Phosphorpentoxid.
Ausbeute: 0,8 g beiges Produkt vom Schmelzpunkt 187-189 ˚C.
$^1$H-NMR (DMSO-$d_6$): δ = 9,10 ppm (s, 1H); 7,05 ppm (d, $^3J_{HH}$ = 8,4 Hz, 2H); 6,67 ppm (d, $^3J_{HH}$ = 8,4 Hz, 2H); 6,57 ppm (d, $^3J_{HH}$ = 8,0 Hz, 1H); 5,91 ppm (d, $^4J_{HH}$ = 2,1 Hz, 1H); 5,79 ppm (dd, $^3J_{HH}$ = 8,0 Hz, $^4J_{HH}$ = 2,1 Hz, 1H); 4,52 ppm (s verbreitert, 4H); 2,60 ppm (m, 2H); 2,00 ppm (m, 2H, Signalüberlagerung mit DMSO).

**Beispiel 5: Herstellung von 1,3-Diamino-4-[2-(4-(dimethylamino)-phenyl)ethyl]-benzol-trihydrochlorid**

10,03 g 4-[(E)-2-(2,4-Dinitrophenyl)ethenyl]-N,N-dimethylanilin, hergestellt nach B. G. Thiemann, S. R. Marder und J. W. Perry, Chem. Mater. 1990, 2, Seite 690-695, werden in 200 ml Ethanol gelöst, und 7 Stunden lang bei 9 bar Wasserstoffdruck an 1 g Pd/C 10%ig hydriert. Anschließend wird der Katalysator durch Filtrieren über Kieselgur entfernt und das Filtrat mit 200 ml 3molarer ethanolischer Salzsäure versetzt. Man konzentriert die erhaltene Lösung bis auf 20% ihres Volumens auf und gibt unter Rühren 600 ml Essigester zu, wobei das Produkt ausfällt. Umkristallisieren aus 100 ml 32%iger Salzsäure ergibt nach dem Trocknen im Vakuum über Phosphorpentoxid 8,4 g farblose Kristalle, die sich oberhalb 250˚C zersetzen.
$^1$H-NMR (DMSO-$d_6$): δ = 7,68 ppm (d, $^3J_{HH}$ = 8,4 Hz, 2H); 7,52 ppm (d, $^3J_{HH}$ = 8.4 Hz, 2H); 7,27 ppm (d, $^3J_{HH}$ = 7,5 Hz, 1H); 7,12 ppm (s, verbreitert, 1H); 6,94 ppm (d verbreitert, $^3J_{HH}$ = 7,5 Hz, 1H); 3,10 ppm (s, 6H); 2,90 ppm (m, 4H).
FAB-MS: 256 $[M+H]^+$, 100%
Elementaranalyse:

$C_{16}H_{21}N_3$, x 2,89HCl x 0,79$H_2O$: M = 374,97

| | %C | %H | %N | %Cl |
|---|---|---|---|---|
| ber.: | 51,25 | 6,85 | 11,21 | 27,32 |
| gef.: | 51,23 | 6,46 | 11,26 | 27,31 |

**Beispiel 6: Herstellung von 1,3-Diamino-4-[2-(4-hydroxyphenyl)-ethyl]-benzol-dihydrochlorid**

Stufe 1: 1-{(E)-2-[4-(Benzyloxy)phenyl]ethenyl}-2,4-dinitrobenzol

18,21 g (0,1 mol) 2,4-Dinitrotoluol, 21,23 g (0,1 mol) 4-Benzyloxybenzaldehyd und 1,8 ml Pyrrolidin werden in 140 ml Dimethylformamid 2 Stunden lang auf 100 ˚C erhitzt, wobei sich die Reaktionsmischung dunkelbraun verfärbt.

Danach läßt man die Reaktionsmischung auf Raumtemperatur abkühlen, verdünnt mit 210 ml Isopropanol und rührt anschließend noch 1 Stunde lang unter Eiskühlung weiter. Man saugt das Produkt ab, wäscht mit wenig kaltem Isopropanol nach und trocknet bei 40 ˚C im Vakuum.
Ausbeute: 21,4 g orangefarbenes Produkt (57% der Theorie).
Elementaranalyse:

$C_{21}H_{16}N_2O_5$, M = 376,37

| | %C | %H | %N |
|---|---|---|---|
| ber. | 6,02 | 4,29 | 7,44 |
| gef. | 67,02 | 4,26 | 7,26 |

**Stufe 2: 1,3-Diamino-4-[2-(4-hydroxyphenyl)ethyl]-benzol-dihydrochlorid**

5,08 g (13,5 mmol) des zuvor beschriebenen Produkts werden in 125 ml Ethanol gelöst, mit 500 mg Pd/C 10%ig versetzt und 4 Stunden lang bei 9 bar Wasserstoffdruck hydriert. Anschließend wird der Katalysator durch Filtrieren über Kieselgur entfernt. Zum Filtrat gibt man unter Rühren 3,7 g 32%ige Salzsäure und engt die Mischung bei 40 ˚C unter vermindertem Druck bis auf die Hälfte des Volumens ein, wobei Kristallisation eintritt. Man saugt das ausgefallene Rohprodukt ab und kristallisiert aus einer Mischung von 15 ml Wasser und 5 ml 32%ige Salzsäure um. Nach Absaugen und Trocknen bei 50 ˚C im Vakuum erhält man 5,4 g beiges Produkt.
$^1$H-NMR (DMSO-$d_6$): δ = 9,33 ppm (s, breit, OH und Wasser); 7,31 ppm (d, $^3J_{HH}$ = 7,2 Hz, 2H); 7,11 ppm (m, 3H); 6,7 ppm (d, $^3J_{HH}$ = 8,2 Hz, 2H); 2,85 ppm (m, 2H); 2,79 ppm (m, 2H).
Elementaranalyse:

$C_{14}H_{16}N_2O$ x 2HCl (0,42% Restfeuchte), M=302,48

| | %C | %H | %N | %Cl |
|---|---|---|---|---|
| ber.: | 55,59 | 6,04 | 9,26 | 23,44 |
| gef.: | 55,80 | 6,00 | 9,30 | 23,20 |

**Beispiel 7: 1,3-Diamino-4-[2-(4-pyridinyl)ethyl]-benzol-trihydrochlorid**

Stufe 1: 4-[(E)-2-(2,4-Dinitrophenyf)ethenyl]pyridin

1,4 g (7,7 mmol) 2,4-Dinitrotoluol, 0,82 g (7,7 mmol) Pyridin-4-carboxaldehyd und 0,2 ml Pyrrolidin werden in einem 10 ml Rundkolben gegeben, der Kolben mit einem Spritzschutz versehen und 20 Sekunden in einem Haushalts-Mikrowellenofen (850 Watt) bestrahlt. Danach läßt man abkühlen und fällt das Produkt durch Zugabe von 20 ml Aceton aus. Absaugen und Trocknen bei 40 ˚C im Vakuum ergeben 0,9 g Produkt (43 % der Theorie).
$^1$H-NMR (DMSO-$d_6$): δ = 8,78 ppm (d, $^4J_{HH}$ = 2,2 Hz, 1H); 8,65 ppm (d, $^3J_{HH}$ = 6,0 Hz, 2H); 8,58 ppm (d, $^3J_{HH}$ = 8,6 Hz, $^4J_{HH}$ = 2,2 Hz, 1H); 8,27 ppm (d, $^3J_{HH}$ = 8,6 Hz, 1H); 8,27 ppm (d, $^3J_{HH}$ = 8,6 Hz, 1H); 7,81 ppm (d, $^3J_{HH}$ = 16,2 Hz, 1H); 7,64 ppm (d, $^3J_{HH}$ = 6,0 Hz, 2H); 7,54 ppm (d, $^3J_{HH}$ = 16,2 Hz, 1H).

**Stufe 2: 1,3-Diamino-4-[2-(4-pyridinyl)ethyl]-benzol-trihydrochlorid**

0,3 g (1 mmol) des Produktes aus Stufe 1 werden in 20 ml Ethanol an 50 mg Pd/C 10%ig 4 Stunden lang bei 9 bar Wasserstoffdruck hydriert. Man filtriert den Katalysator über Kieselgur ab und gibt 10 ml einer 3,3molaren ethanolischen Salzsäurelösung zu. Zur Isolierung des Produktes dampft man bei 40 ˚C unter reduziertem Druck weitgehend ein und fällt das Salz durch Zugabe von 10 bis15 ml Essigsäureethylester aus. Nach dem Absaugen und Trocknen im Vakuum bei 50 ˚C erhält man 0,28 g beiges Produkt.
$^1$H-NMR (DMSO-$d_6$): δ = 8,88 ppm (d, $^3J_{HH}$ = 6,5 Hz, 2H); 8,11 ppm (d, $^3J_{HH}$ = 6,5 Hz, 2H); 7,25 ppm (d, $^3J_{HH}$ = 8,0 Hz, 1H); 7,09 ppm (s, verbreitert, 1H); 6,92 ppm (d, verbreitert, $^3J_{HH}$ = 8,0 Hz, 1H); 3,24 ppm (m, 2H); 3,00 ppm (m, 2H).

**Beispiel 8: 1,3-Diamino-4-[2-(3-pyridinyl)ethyl]-benzol-trihydrochlorid**

In Analogie zu der im vorstehenden Beispiel 7 beschriebenen Synthese erhält man aus Pyridin-3-carboxaldehyd bei 48% Gesamtausbeute das entsprechende 1,3-Diamino-4-[2-(3-pyridinyl)ethyl]-benzol-trihydrochlorid.
$^1$H-NMR (DMSO-$d_6$): δ = 9,04 ppm (s, 1H); 8,83 ppm (d, $^3J_{HH}$ = 5,4 Hz, 1H); 8,69 ppm (d, $^3J_{HH}$ = 8,1 Hz, 1H); 8,07 ppm

(dd, $^4J_{HH}$ = 5,4 Hz, $^3J_{HH}$ = 8,1 Hz, 1H); 7,39 ppm (d, $^3J_{HH}$ = 8,0 Hz, 1H); 7,33 ppm (s, 1H); 7,15 ppm (d, $^3J_{HH}$ = 8,0 Hz, 1H); 3,20 ppm (m, 2H); 3,04 ppm (m, 2H).

Elementaranalyse:

$C_{13}H_{15}N_3$ x 3HCl (0,89% Restfeuchte), M = 325,55

| | %C | %H | %N | %Cl |
|---|---|---|---|---|
| ber.: | 47,96 | 5,67 | 12,91 | 32,67 |
| gef.: | 47,60 | 5,20 | 12,90 | 32,50 |

**Beispiel 9: Oxidationshaarfärbemittel, Lösung basisch**

In 10 ml der nachfolgend angegebenen Basisrezeptur werden jeweils 0,25 mmol der in Tabelle 1 genannten erfindungsgemäßen m-Diaminobenzole (1a- 1 v) gelöst.

**Basisrezeptur**

| | |
|---|---|
| 80,00 g | Ethanol |
| 100,00 g | Natriumlaurylethersulfat, 28%ige wässrige Lösung |
| 90,00 g | Ammoniak, 25%ige wässrige Lösung |
| 3,00 g | Ascorbinsäure |
| 4,00 g | Natriumsulfit |
| ad 1000,00 g | Wasser, entmineralisiert |

In gleicher Weise werden jeweils 0,25 mmol der in Tabelle 1 genannten Entwicklersubstanzen in 10 ml der Basisrezeptur gelöst.

Unmittelbar vor der Anwendung werden jeweils 10 g Entwicklerlösung mit 10g Kupplerlösung miteinander vermischt. Zu dieser Mischung gibt man anschließend 20 g einer 6%igen Wasserstoffperoxid-Lösung und erhält nach dem Durchmischen die gebrauchsfertigen Färbelösungen.

Die so erhaltenen gebrauchsfertigen Oxidationshaarfärbemittel werden anschließend auf gebleichte Haare aufgetragen. Nach einer Einwirkzeit von 30 Minuten bei 40°C werden die Haarsträhnen ausgespült, mit einem Farbpflegeshampoo gewaschen und getrocknet.

Die erhaltenen Färbungen werden mit einem Minolta-Chromameter CR-300 im L*a*b*-Farbsystem vermessen, wobei

L* für die Helligkeit; +a* für den Rotanteil; -a* für den Grünanteil; +b* für den Gelbanteil und -b* für den Blauanteil steht.

In den folgenden Tabellen stehen die ΔE-Werte für Farbveränderungen im L*a*b*-System und werden nach der Gleichung

$$\Delta E = \sqrt{(L_i - L_o)^2 + (a_i - a_o)^2 + (b_i - b_o)^2}$$

berechnet. Darin stellen $L_0$, $a_0$ und $b_0$ Meßwerte vor und $L_i$, $a_i$ und $b_i$ die entsprechenden Werte nach den Waschversuchen dar (zur Erläuterung: je höher der errechnete ΔE-Wert ist, desto höher ist der Farbverlust bzw. die Farbtonänderung).

Die erfindungsgemäßen m-Diaminobenzole sind in der Lage, mit p-Phenylendiaminen sehr reine stahlblaue Ausfärbungen zu liefern, die einen sehr geringen Rotanteil besitzen. Im L*a*b*-Farbraum erkennt man dies an sehr niedrigen +a*-Werten sowie den hohen Beträgen für -b*.

Die entsprechenden L*a*b*-Werte sind in Tabelle 1 zusammengefaßt.

Tabelle 1

| Entwickler / Kuppler | 1,4-Diamino-2-methyl-benzol-sulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl)amino)-anilin-sulfat(1:1) |
|---|---|---|---|---|---|
| 2,4-Diamino-1-(2'-hydroxy-ethoxy)-benzol [Vergleichs-substanz] | L= 21,68<br>a= 4,88<br>b= -12,99 | L= 21,72<br>a= 6,28<br>b= -16,51 | L= 44,73<br>a= 17,91<br>b= 3,90 | L= 23,48<br>a= 37,90<br>b= 7,18 | L= 22,47<br>a= 4,29<br>b= -22,26 |
| 1b | L= 22,03<br>a= 5,75<br>b= -21,91 | L= 25,00<br>a= 5,10<br>b= -24,69 | L= 59,60<br>a= 13,03<br>b= -4,43 | L= 25,00<br>a= 40,51<br>b= -12,28 | / |
| 1g | L= 28,57<br>a= 2,92<br>b= -22,58 | L= 32,50<br>a= 2,80<br>b= -23,33 | L= 66,71<br>a= 7,52<br>b= 1,39 | L= 34,09<br>a= 43,50<br>b= -10,62 | L= 33,26<br>a= -2,41<br>b= -24,29 |

| Entwickler / Kuppler | 1,4-Diamino-2-methyl-benzol-sulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl)amino)-anilin-sulfat(1:1) |
|---|---|---|---|---|---|
| 1d | L= 26,60<br>a= 0,03<br>b= -15,98 | L= 31,62<br>a= 0,43<br>b= -19,48 | L= 62,35<br>a= 7,29<br>b= 1,76 | L= 28,61<br>a= 37,74<br>b= -11,98 | L= 26,68<br>a= -3,56<br>b= -16,76 |
| 1h | L= 22,12<br>a= 2,62<br>b= -16,80 | L= 24,99<br>a= 3,81<br>b= -22,88 | L= 53,89<br>a= 3,06<br>b= -3,49 | L= 24,72<br>a= 38,28<br>b= -8,78 | L= 27,32<br>a= 0,14<br>b= -25,20 |
| 1e | L= 21,92<br>a= 6,45<br>b= -23,49 | L= 26,36<br>a= 4,70<br>b= -22,85 | L= 58,86<br>a= 10,47<br>b= -2,20 | L= 20,02<br>a= 41,12<br>b= -9,21 | L= 26,46<br>a= 0,74<br>b= -25,38 |
| 1i | L= 23,79<br>a= 5,45<br>b= -24,91 | L= 24,50<br>a= 5,01<br>b= -23,66 | L= 59,04<br>a= 10,19<br>b= 1,42 | L= 22,31<br>a= 38,08<br>b= -8,13 | L= 27,42<br>a= 1,06<br>b= -26,11 |

| Entwickler / Kuppler | 1,4-Diamino-2-methyl-benzol-sulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl)amino)-anilin-sulfat(1:1) |
|---|---|---|---|---|---|
| 1k | L= 25,20<br>a= 4,31<br>b= -24,00 | L= 27,83<br>a= 4,71<br>b= -25,92 | L= 58,73<br>a= 4,72<br>b= -1,70 | L= 23,59<br>a= 37,59<br>b= -11,00 | L= 29,29<br>a= -3,08<br>b= -20,76 |
| 1v | L= 25,20<br>a= 4,31<br>b= -24,00 | L= 27,83<br>a= 4,71<br>b= -25,92 | L= 58,73<br>a= 4,72<br>b= -1,70 | L= 23,59<br>a= 37,59<br>b= -11,00 | L= 29,29<br>a= -3,08<br>b= -20,76 |
| 1a | L= 26,46<br>L= 5,11<br>a= -26,36 | L= 27,02<br>a= 4,34<br>b= -24,72 | L= 73,26<br>a= -0,28<br>b= 8,36 | L= 25,64<br>a= 38,97<br>b= -9,67 | L= 29,27<br>a= 1,12<br>b= -28,11 |
| 1d' | L= 29,11<br>a= 2,15<br>b= -22,47 | L= 29,82<br>a= 2,62<br>b= -23,89 | L= 65,71<br>a= 1,91<br>b= -0,34 | L= 27,26<br>a= 38,87<br>b= -14,47 | L= 29,22<br>a= -0,62<br>b= -25,21 |

| Entwickler / Kuppler | 1,4-Diamino-2-methyl-benzol-sulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl)amino)-anilin-sulfat(1:1) |
|---|---|---|---|---|---|
| 1q | L= 24,33<br>a= 4,91<br>b= -24,38 | L= 27,23<br>a= 3,41<br>b= -23,93 | L= 63,91<br>a= 0,59<br>b= -0,15 | L= 23,85<br>a= 36,41<br>b= -13,85 | L= 27,74<br>a= 0,85<br>b= -26,02 |
| 1l | L= 54,12<br>a= -2,01<br>b= -1,76 | L= 56,91<br>a= -1,15<br>b= -1,36 | L= 82,60<br>a= 1,92<br>b= 12,12 | L= 50,29<br>a= 29,30<br>b= -6,24 | L= 49,59<br>a= -6,65<br>b= -8,27 |
| 1m | L= 48,28<br>a= -2,77<br>b= -7,68 | L= 49,26<br>a= -1,98<br>b= -7,54 | L= 78,88<br>a= 4,03<br>b= 7,72 | L= 47,11<br>a=34,56<br>b= -7,95 | L= 46,24<br>a= -7,33<br>b= -11,39 |
| 1n | L= 24,51<br>a= 4,10<br>b= -23,11 | L= 25,38<br>a= 4,28<br>b= -23,95 | L= 57,89<br>a= 4,10<br>b= -3,42 | L= 24,82<br>a= 37,96<br>b= -11,46 | L= 27,64<br>a= -0,44<br>b= -23,88 |

| Entwickler / Kuppler | 1,4-Diamino-2-methyl-benzol-sulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl)amino)-anilin-sulfat(1:1) |
|---|---|---|---|---|---|
| 1o | L= 31,04<br>a= 2,05<br>b= -23,32 | L= 31,58<br>a= 3,21<br>b= -24,12 | L= 67,72<br>a= 6,56<br>b= 0,47 | L= 30,01<br>a= 43,21<br>b= -8,82 | L= 32,42<br>a= -2,94<br>b= -25,13 |
| 1p | L= 23,90<br>a= 5,17<br>b= -24,35 | L= 24,91<br>a= 6,00<br>b= -26,23 | L= 72,31<br>a= 7,76<br>b= 8,77 | L= 29,90<br>a= 43,20<br>b= -13,71 | L= 33,40<br>a= -2,36<br>b= -27,17 |
| 1s | L= 43,51<br>a= -0,91<br>b= -5,06 | L= 49,73<br>a= -2,18<br>b= -2,52 | L= 77,54<br>a= 2,63<br>b= 9,10 | L= 45,77<br>a= 30,98<br>b= -6,44 | L= 46,05<br>a= -8,49<br>b= -3,19 |
| 1r | L= 0,33<br>a= -2,86<br>b= -1,19 | L= 42,21<br>a= -2,66<br>b= -8,26 | L= 72,40<br>a= 1,53<br>b= 5,37 | L= 40,15<br>a= 35,05<br>b= -8,12 | L= 40,09<br>a= -8,78<br>b= -9,23 |

| Entwickler / Kuppler | 1,4-Diamino-2-methyl-benzol-sulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl)amino)-anilin-sulfat(1:1) |
|---|---|---|---|---|---|
| 1t | L= 24,85<br>a= 0,87<br>b= -15,51 | L= 26,89<br>a= 2,45<br>b= -19,78 | L= 58,62<br>a= 6,77<br>b= -2,73 | L= 25,46<br>a= 38,82<br>b= -10,57 | L= 29,12<br>a= -2,05<br>b= -21,51 |
| 1u | L= 44,71<br>a= -1,61<br>b= 1,29 | L= 48,76<br>a= -1,73<br>b= 4,89 | L= 74,48<br>a= 1,72<br>b= 7,50 | L= 74,03<br>a= 1,20<br>b= -7,52 | L= 40,77<br>a= -6,60<br>b= -6,63 |
| 1f | L= 39,76<br>a= -2,40<br>b= -7,08 | L= 41,28<br>a= -1,68<br>b= -12,76 | L= 68,65<br>a= 7,25<br>b= 2,68 | L= 40,29<br>a= 40,36<br>b= -8,09 | L= 37,57<br>a= -6,89<br>b= -12,46 |
| 1c | L= 25,82<br>a= -0,10<br>b= -16,22 | L= 30,90<br>a= 1,80<br>b= -22,58 | L= 67,13<br>a= 5,85<br>b= 4,74 | L= 43,78<br>a= 33,22<br>b= -8,04 | L= 45,19<br>a= -6,25<br>b= -21,11 |

**Beispiel 10: Oxidationshaarfärbemittel, Creme basisch**

| | |
|---|---|
| 15,00 g | Cetylstearylalkohol (50/50) |
| 5,00 g | Glycerinmonostearat |
| 2,00 g | Cocamid DEA |
| 10,00 g | Natriumlaurylethersulfat, 28%ige wäßrige Lösung |
| 0,30 g | Ascorbinsäure |
| 0,40 g | Natriumsulfit |
| 4,50 g | Ammoniak, 25%ige wäßrige Lösung |
| 0,25 mmol | Entwickler gemäß Tabelle 2 |
| 0,25 mmol | Kuppler gemäß Tabelle 2 |
| ad 100,00 g | Wasser, entmineralisiert |

Der pH-Wert der Cremes liegt zwischen 10 und 10,5.
Unmittelbar vor der Anwendung wird die Creme mit 100 Gramm einer 6%igen wäßrigen Wasserstoffperoxidlösung vermischt und das erhaltene gebrauchsfertige Oxidationshaarfärbemittel in der notwendigen Menge auf gebleichte Haare aufgetragen. Der pH-Wert der gebrauchsferigen Mischungen liegt zwischen 9,5 und 10. Nach einer Einwirkzeit von 30 Minuten bei 40 ˚C wird mit einem Farbpflegeshamopoo gewaschen, gespült und getrocknet.
Die erhaltenen Farbnuancen und Farbintensitäten sind in Tabelle 2 zusammengefaßt.

Tabelle 2: Färbeergebnisse aus Cremeformulierung

| Entwickler<br><br>Kuppler | 1,4-Diamino-2-methyl-benzolsulfat(1:1) | 1,4-Diamino-2-(2-hydroxyethyl)-benzol-sulfat(1:1) | 4-Amino-3-methyl-phenol | 4,5-Damino-1-(2-hydroxyethyl)-1H-pyrazol-sulfat(1:1) | 4-(Di(2-hydroxyethyl) amino)-anilin-sulfat(1:1) |
|---|---|---|---|---|---|
| 1a | L= 17,0<br>a= 3,6<br>B=-11,7<br>dunkelblau | L= 18,0<br>a= 4,8<br>b= -15,7<br>dunkelblau | L= 37,4<br>a= 7,3<br>b= -10,6<br>rose | L= 18,4<br>a= 24,1<br>b= -5,9<br>brillantviolett | L= 20,2<br>a= 2,8<br>b= -18,2<br>dunkel stahlblau |
| 1g | L= 18,1<br>a= 3,9<br>b= -14,9<br>dunkelblau | L= 20,3<br>a= 4,1<br>b= -17,1<br>dunkelblau | L= 44,8<br>a= 6,1<br>b= -7,1<br>rose | L= 19,2<br>a= 29,1<br>b= -5,9<br>brillantviolett | L= 19,7<br>a= 2,2<br>b=-17,1<br>dunkel stahlblau |
| 1e | L= 21,6<br>a= -0,2<br>b= -12,1<br>dunkelblau | L= 20,9<br>a= 2,9<br>b= -16,5<br>dunkelblau | L= 49,9<br>a= 7,5<br>b= -5,0<br>hellrosé | L= 20,4<br>a= 32,5<br>b= -7,6<br>brillantviolett | L= 21,3<br>a= -0,8<br>b= -14,9<br>dunkelcyan |

**Beispiel 11: Waschbeständigkeit**

Gefärbte Haarsträhnen aus Beispiel 10 werden je fünfmal 1 Minute lang shampooniert und getrocknet und nach der Schlußtrocknung erneut im L*a*b*-System vermessen.Die Unterschiede in den L*a*b*-Werten sowie die daraus resultierenden ΔE-Werte sind in den Tabellen 3a bis 3c zusammengefaßt.

Tabelle 3a

| Entwickler / Kuppler | 1,4-Diamino-2-methyl-benzol-sulfat(1:1) | | | | |
|---|---|---|---|---|---|
| 1a | | L | a | b | ΔE |
| | gefärbt: | 17,0 | 3,6 | -11,7 | |
| | gewaschen: | 17,2 | 3,2 | -11,3 | 0,6 |

Tabelle 3b

| Entwickler / Kuppler | 4-Amino-3-methyl-phenol | | | | |
|---|---|---|---|---|---|
| 1g | | L | a | b | ΔE |
| | gefärbt: | 44,8 | 6,1 | -7,1 | |
| | gewaschen: | 47,3 | 6,0 | -5,4 | 3,0 |

Tabelle 3c

| Entwickler / Kuppler | 4,5-Damino-1-(2-hydroxyethyl)-1H-pyrazol-sulfat(1:1) | | | | |
|---|---|---|---|---|---|
| 1e | | L | a | b | ΔE |
| | gefärbt: | 20,4 | 32,5 | -7,6 | |
| | gewaschen: | 20,9 | 32,4 | -9,1 | 1,6 |

Wie die ΔE-Werte zeigen, liegen die Unterschiede nach den Wasch- und Trockenzyklen im Bereich der Meßtoleranzen uns sind visuell praktisch nicht sichtbar.

**Beispiel 12: Oxidationshaarfärbemittel mit Direktzieher (basisch)**

| | |
|---|---|
| 32,20 g | Cetylstearylalkohol + Ceteareth20 |
| 10,12 g | Glycerinstearat, pflanzlich (Tegin TB der Fa.Th. Goldschmidt) |
| 3,68 g | Lanolinalkohol |
| 4,00 g | Glykoldistearat |
| 5,60 g | Natriumlaurylethersulfat |

(fortgesetzt)

| | |
|---|---|
| 0,92 g | Sodium-cocoyl-isethionate |
| 0,20 g | Ethylendiaminotetraacetat |
| 0,60 g | Ascorbinsäure |
| 0,80 g | Natriumsulfit |
| 0,38 g | 1,4-Diamino-2-methylbenzol-sulfat (1:1) |
| 0,66 g | 1,4-Diamino-2-(2-hydroxyethyl)-benzol-sulfat (1:1) |
| 0,46 g | 4,5-Damino-1-(2-hydroxyethyl)-1H-pyrazol-sulfat(1:1) |
| 0,54 g | 4-Amino-3-methyl-phenol |
| 0,54 g | 1,3-Diamino-4-(2-phenylethyl)-benzol-dihydrochlorid (1a) |
| 0,26 g | 1,3-Diamino-benzol |
| 0,36 g | 3-Amino-phenol |
| 0,42 g | 5-Amino-2-methyl-phenol |
| 0,60 g | 2-Chlor-6-(ethylamino)-4-nitro-phenol |
| 9,00 g | Ammoniak, 25%ige wäßrige Lösung |
| ad 200,00 g | Wasser, entmineralisiert |

Der pH-Wert der Creme beträgt 9,8.
Unmittelbar vor der Anwendung wird mit 100 Gramm einer 6%igen bzw, 9%igen wäßrigen Wasserstoffperoxidlösung vermischt. Die so erhaltenen gebrauchsferigen Oxidationshaarfärbemittel weisen pH-Wert von 9,4 bzw, 9,2 auf. Die gebrauchsferigen Oxidationshaarfärbemittel werden in der für eine Färbung ausreichenden Menge auf das Haar aufgetragen und nach einer Einwirkzeit von 30 Minuten bei 40˚C mit Wasser ausgespült, mit einem Farbpflegeshampoo gewaschen, gespült und getrocknet.
Die erhaltenen L*a*b*-Werte und Farbnuancen sind in Tabelle 4 zusammengefaßt.

Tabelle 4: Färbungen bei unterschiedlichen Haartypen und Wasserstoffperoxid-Konzentrationen

| **Haartyp** | **Färbemischung I** (Wasserstoffperoxid 6%) | | | | **Färbemischung II** (Wasserstoffperoxid 9%) | | | |
|---|---|---|---|---|---|---|---|---|
| Tierhaar gebleicht | | L | a | b | | L | a | b |
| | ungefärbt: | 82,5 | -0,2 | 11,0 | ungefärbt: | 81,1 | -0,3 | 10,3 |
| | gefärbt: | 17,6 | 3,4 | 2,6 | gefärbt: | 18,5 | 3,9 | 3,1 |
| Humanhaar mit 50% Grauanteil | | L | a | b | | L | a | b |
| | ungefärbt: | 54,7 | 0,8 | 12,1 | ungefärbt: | 58,9 | 0,2 | 12,3 |
| | gefärbt: | 21,8 | 6,5 | 5,0 | gefärbt: | 21,0 | 6,8 | 4,0 |
| Humanhaar mittelblond, Tontiefe 7/0 | | L | a | b | | L | a | b |
| | ungefärbt: | 31,6 | 5,9 | 11,2 | ungefärbt: | 29,5 | 5,5 | 10,2 |
| | gefärbt: | 20,7 | 5,1 | 3,8 | gefärbt: | 20,1 | 5,1 | 4,0 |

Alle Haare sind in einem dunklen Aubergineton gefärbt, Bei dem Humanhaar, das ungefärbt einen Grauanteil von 50% hatte, erfolgte eine vollständige Grauabdeckung und eine völlig einheitliche Färbung.
Signifikante Unterschiede, die durch die Verwendung von höheren Anteilen an Wasserstoffperoxid zurückzuführen wären, sind nicht festzustellen.

Alle Prozentangaben stellen, soweit nicht anders angegeben, Gewichtsprozente dar.

## Patentansprüche

1. m-Diaminobenzol, welches ausgewählt ist aus der Gruppe bestehend aus 1,3-Diamino-4-(2-phenylethyl)-benzol-dihydrochlorid, 1,3-Diamino-4-[2-(4-methylphenyl)-ethyl]-benzol, 1,3-Diamino-4-[2-(1-naphthyl)ethyl]-benzol-dihydrochlorid, 1,3-Diamino-4-[2-(4-methoxyphenyl)ethyl]-benzol-dihydrochlorid,1,3-Diamino-4-[2-(4-methoxyphenyl) ethyl]-benzol, 1,3-Diamino-4-[2-(4-hydroxyphenyl)ethyl]-benzol-dihydrochlorid, 1,3-Diamino-4-[2-(2-hydroxyphenyl)ethyl]-benzol, 1,3-Diamino-4-[2-(4-(dimethylamino)-phenyl)ethyl]-benzol-trihydrochlorid, 4-[2-(4-Cyanophenyl)ethyl]-1,3-diamino-benzol-dihydrochlorid, 1,3-Diamino-4-[2-(4-pyridinyl)ethyl]-benzol-trihydrochtorid, 1,3-Diamino-4-[2-(3-pyridinyl)ethyl]-benzol-trihydrochlorid, 1,3-Diamino-4-[2-(4-biphenyl)ethyl]-benzol-dihydrochlorid, 1,3-Diamino-4-[2-(2,4-dimethoxyphenyl)ethyl]-benzol, 1,3-Diamino-4-[2-(4-hydroxy-3-methoxyphenyl)ethyl]-benzol, 1,3-Diamino-4-[2-(3,4,5-trihydroxyphenyl)-ethyl]-benzol, 1,3-Diamino-4-[2-(4-(2-hydroxyethoxy)phenyl)ethyl]-benzol, 1,3-Diamino-4-[2-(2-thienyl)ethyl]-benzol, 1,3-Diamino-4-[2-(2-furyl)ethyl]-benzol, 1,3-Diamino-4-[2-(5-methyl-2-furyl)ethyl]-benzol, 1,3-Diamino-4-[2-(1H-indol-3-yl)ethyl]-benzol, 1,3-Diamino-4-[2-(-methyl-1H-indol-3-yl)ethyl]-benzol und 1,3-Diamino-4-[2-(2-pyridinyl)ethyl]-benzoltrihydrochlorid.

2. Mittel zur oxidativen Färbung von Fasern, **dadurch gekennzeichnet, dass** es in einer geeigneten Farbträgermasse mindestens ein m-Diaminobenzol nach Anspruch 1 enthält.

3. Mittel gemäß Anspruch 2, **dadurch gekennzeichnet, dass** es das m-Diaminobenzol in einer Menge von 0,01 bis 20 Gewichtsprozent enthält.

4. Mittel gemäß Anspruch 2 oder 3, **dadurch gekennzeichnet, dass** es zusätzlich mindestens eine Entwicklersubstanz und/oder eine zusätzliche Kupplersubstanz und/oder einen direktziehenden Farbstoff enthält.

5. Mittel gemäß einem der Ansprüche 2 bis 4, **dadurch gekennzeichnet, dass** es vor der Anwendung mit einem Oxidationsmittel vermischt wird.

6. Mittel gemäß Anspruch 5, **dadurch gekennzeichnet, dass** das Oxidationsmittel Wasserstoffperoxid ist.

7. Mittel gemäß einem der Ansprüche 2 bis 6, **dadurch gekennzeichnet, dass** es ein Haarfärbemittel ist.

8. Verwendung der m-Diaminobenzole nach Anspruch 1 zur oxidativen Färbung von Fasern.

## Claims

1. m-Diaminobenzene, which is selected from the group consisting of 1,3-diamino-4-(2-phenylethyl)-benzene-dihydrochloride, 1,3-diamino-4-[2-(4-methylphenyl)-ethyl]-benzene, 1,3-diamino-4-[2-(1-naphthyl)ethyl]-benzene-dihydrochloride, 1,3-diamino-4-[2-(4-methoxyphenyl)ethyl]-benzene-dihydro chloride,1,3-diamino-4-[2-(4-methoxyphenyl)ethyl]-benzene, 1,3-diamino-4-[2-(4-hydroxyphenyl)ethyl]-benzene-dihydrochloride, 1,3-diamino-4-[2-(2-hydroxyphenyl)ethyl]-benzene, 1,3-diamino-4-[2-(4-(dimethylamino)-phenyl)ethyl]-benzene-trihydrochloride, 4-[2-(4-cyanophenyl)ethyl]-1,3-diamino-benzene-dihydrochloride, 1,3-diamino-4-[2-(4-pyridinyl)ethyl]-benzene-trihydrochloride, 1,3-diamino-4-[2-(3-pyridinyl)ethyl]-benzene-trihydrochloride, 1,3-diamino-4-[2-(4-biphenyl)ethyl]-benzene-dihydrochloride, 1,3-diamino-4-[2-(2,4-dimethoxyphenyl)ethyl]-benzene, 1,3-diamino-4-[2-(4-hydroxy-3-methoxyphenyl)ethyl]-benzene, 1,3-diamino-4-[2-(3,4,5-trihydroxyphenyl)-ethyl]-benzene, 1,3-diamino-4-[2-(4-(2-hydroxyethoxy)phenyl)ethyl]-benzene, 1,3-diamino-4-[2-(2-thienyl)ethyl]-benzene, 1,3-diamino-4-[2-(2-furyl) ethyl]-benzene, 1,3-diamino-4-[2-(5-methyl-2-furyl)ethyl]-benzene, 1,3-diamino-4-[2-(I H-indol-3-yl)ethyl] -benzene, 1,3-diamino-4-[2-(1-methyl-1H-indol-3-yl)ethyl]-benzene, and 1,3-diamino-4-[2-(2-pyridinyl)ethyl]-benzene-trihydrochloride.

2. An agent for the oxidative dyeing of fibers, **characterized in that** it contains at least one m-diaminobenzene according to Claim 1 in a suitable dye medium.

3. The agent according to Claim 2, **characterized in that** it contains the m-diaminobenzene in an amount of from 0.01 to 20 percent by weight.

4. The agent according to Claim 2 or 3, **characterized in that** it additionally contains at least one developer substance and/or an additional coupler substance and/or a direct dye.

5. The agent according to any one of Claims 2 to 4, **characterized in that** it is mixed with an oxidizing agent prior to application.

6. The agent according to Claim 5, **characterized in that** the oxidizing agent is hydrogen peroxide.

7. The agent according to any one of Claims 2 to 6, **characterized in that** it is a hair dye.

8. The use of m-diaminobenzene according to Claim 1 for the oxidative dyeing of fibers.

## Revendications

1. m-Diaminobenzène, qui est choisi parmi le groupe comprenant 1,3-diamino-4-(2-phényléthyl)-benzène-dichlorhydrate, 1,3-diamino-4-[2-(4-méthylphényl)-éthyl]-benzène, 1,3-diamino-4-[2-(1-naphtyl)éthyl]-benzène-dichlorhydrate, 1,3-diamino-4-[2-(4-méthoxy-phényl)éthyl]-benzène-dichlorhydrate, 1,3-diamino-4-[2-(4-méthoxyphényl) éthyl] -benzène, ,1,3 -diamino-4-[2-(4-hydroxyphényl)éthyl] -benzène-dichlorhydrate, 1,3-diamino-4-[2-(2-hydroxyphényl)éthyl]-benzène, 1,3-diamino-4-[2-(4-(diméthylamino)-phényl)éthyl]-benzène-trihydrochlorhydrate, 4-[2-(4-cyanophényl)éthyl]-1,3-diamino-benzène-dichlorhydrate, 1,3-diamino-4-[2-(4-pyridinyl)éthyl]-benzène-trihydrochlorhydrate, 1,3-diamino-4-[2-(3-pyridinyl)éthyl]-benzène-trihydrochlorhydrate, 1,3 -diamino-4-[2-(4-biphényl) éthyl]-benzène-dichlorhydrate, 1,3-diamino-4-[2-(2,4-diméthoxyphényl)éthyl]-benzène, 1,3-diamino-4-[2-(4-hydroxy-3-méthoxyphényl)éthyl]-benzène, 1,3-diamino-4-[2-(3,4,5-trihydroxyphenyl)-éthyl]-benzène, 1,3-diamino-4-[2-(4-(2-hydroxyéthoxy)phényl)éthyl]-benzène, 1,3-diamino-4-[2-(2-thiényl)éthyl]-benzène, 1,3-diamino-4-[2-(2-furyl)éthyl] -benzène 1,3-diamino-4-[2-(5-méthyl-2-furyl)éthyl]-benzène, 1,3-diamino-4-[2-(1H-indol-3-yl)éthyl]-benzène, 1,3-diamino-4-[2-(1-méthyl-1H-indol-3-yl)éthyl]-benzène, et 1,3-diamino-4-[2-(2-pyridinyl)éthyl]-benzène-trihydrochlorhydrate.

2. Agent pour la teinture oxydative de fibres, **caractérisé en ce qu'**il contient au moins un m-diaminobenzène selon la revendication 1 dans un véhicule de teinture approprié.

3. Agent selon la revendication 2, **caractérisé en ce qu'**il contient le m-diaminobenzène en une quantité allant de 0,01 à 20 pour cent en poids.

4. Agent selon la revendication 2 ou 3, **caractérisé en ce qu'**il contient, en outre, au moins une substance de type développeur et/ou une substance de type copulant supplémentaire et/ou une teinture directe.

5. Agent selon l'une quelconque des revendications 2 à 4, **caractérisé en ce qu'**il est mélangé avec un agent oxydant avant application.

6. Agent selon la revendication 5, **caractérisé en ce que** l'agent oxydant est du peroxyde d'hydrogène.

7. Agent selon l'une quelconque des revendications 2 à 6, **caractérisé en ce qu'**il est une teinture capillaire.

8. Utilisation de m-diaminobenzène selon la revendication 1 pour la teinture oxydative de fibres.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6461389 B1


### In der Beschreibung aufgeführte Nicht-Patentliteratur


- **S. B Lokhande ; D. W. Rangnekar.** *Ind. J. Chem.,* 1986, vol. 25B, 485-8
- **B. G. Tiemann.** *Chem. Mater,* 1990, vol. 2, 690-695
- **S. B Lokhande ; D. W. Rangnekar.** *Ind. J. Chem.,* 1986, vol. 25B, 485-488
- **S. Saravanan ; P. Srinivasan.** *Synth. Commun.,* 2001, vol. 31 (6), 823-826
- **B. G. Thiemann ; S. R. Marder ; J. W. Perry.** *Chem. Mater.,* 1990, vol. 2, 690-695